# EUROPEAN PATENT SPECIFICATION

(11) **EP 0 812 176 B1**
(45) Date of publication and mention of the grant of the patent: **29.12.1999**
(21) Application number: 96904372.8
(22) Date of filing: 26.02.1996
(51) Int. Cl.: A61J 1/00, A61J 1/05, A61M 5/162, A61M 39/14

(54) **SYSTEM FOR ENTERAL/PARENTERAL ADMINISTRATION OF A SUBSTANCE**
ENTERAL/PARENTERAL VERABREICHUNGSSYSTEM FÜR WIRKSTOFFE
SYSTEME POUR L'ADMINISTRATION ENTERALE/PARENTERALE D'UNE SUBSTANCE

(30) Priority: 27.02.1995 NL 9500382
(43) Date of publication of application: 17.12.1997
(73) Proprietor: N.V. Nutricia, 2700 MA Zoetermeer (NL)
(72) Inventor: KUPER, Tom, Gerrit, NL-8264 BX Kampen (NL)
(74) Representative: de Bruijn, Leendert C.
(86) International application number: NL9600093
(87) International publication number: WO9626701

(56) References cited:
- EP-A- 0 258 579
- DE-A- 2 026 086
- DE-B- 1 288 751
- NL-C- 88 253
- US-A- 3 127 892
- US-A- 5 334 180

## Description

The invention relates to a system for the administration of a medical substance, provided with a metering line system for connection to a patient, a substance reservoir which has a metering opening with a sealing element to protect the interior of the reservoir from the environment and a connecting section to connect the reservoir via the metering opening to the metering line system, which connecting section defines a medium connection which is essentially hermetically screened from the environment when the sealing element is broken and comprises a flexible channel section, which can be shortened by deformation, penetration means, which are located in the channel section and can be pushed through the sealing element on shortening of the channel section and a relatively rigid coupling element, connected to the reservoir.

Such a system is known from Dutch patent NL-C-88253. In this document a liquid reservoir is provided with a flexible tube part which is connected to a metal tube section comprising a rigid metal collar. Around this collar a coupling bush is rotatably mounted. Inside the flexible tube part, a hollow needle is mounted which can be manually pushed through a cooling gap on the reservoir tube part. With the known system the reservoir can be efficiently and aseptically coupled to an injection device.

The known system has as a disadvantage that during storage or transportation, the needle may inadvertently puncture the reservoir. Furthermore, the connecting system which is attached to the reservoir is relatively complex, thus increasing the cost of the reservoir and increasing the amount of waste which is produced upon discarding the reservoir.

Furthermore, the sealing between the reservoir and the end of the tube of the known injection device is relatively complex.

US-A 5 334 180 discloses a system in which the reservoir is a bag of flexible film which contains a sterile fluid. An essentially non-deformable tube stub protrudes from the bottom edge of the bag. A cylindrical, chamfered blade is located in said tube stub, the cutting edge of said blade facing towards the bag. From the outside, a pin, which extends through the tube stub, can be made to engage with the cylindrical blade for puncturing the bag open by hand. This puncturing open must be carried out before the line system is connected to the bag.

Because the line system can be connected to said bag in a manner suitable for metering (in particular leak-free) only after the bag has been punctured open, there are risks of spilling the contents, and a risk of contamination from the outside, during the puncturing operation. The known system is consequently difficult to handle. For example, it is necessary to hold the bag the wrong way up during puncturing. Only after the line system has been connected to the bag after puncturing can the bag be suspended in its operating position. This not only makes it difficult to prepare the bag and the line system for use, but it is only possible to check that the line system has been correctly connected to the bag after the bag has already been punctured open.

German Offenlegungsschrift 2 026 086 discloses a system wherein blood is stored in a flexible bag which is provided on the underside with a tube section with a seal. A flexible tube is connected to the sealing tube section via a shrink sleeve. A needle is present in the interior of the flexible tube, which needle can be pushed through the seal of the bag by compressing the flexible tube. The disadvantage of the known system is that the line system and the flexible bag have to be permanently connected to one another. The known blood transfusion systems are consequently relatively difficult to store and to transport. Furthermore, if several bags of blood have to be administered during the treatment, a completely new line system will always have to be used for every bag. This inefficient use of line systems gives rise to a relatively large amount of waste, which is problematical from the standpoint of waste management. Furthermore, the system having a fixed line system is insufficiently flexible if reservoirs of differing capacity, such as, for example, 0.5 litre, 1 litre and 1.5 litre, have to be used. Finally, the sterilisation of the combination of reservoirs with a line system firmly connected thereto presents problems since not all materials are able to withstand sterilisation by means of high temperatures in an autoclave. When designing the known systems, the materials have to be matched to one another so that joint sterilisation is possible.

A reservoir for use in a system for the administration of a medical substance as discussed above, which reservoir is provided with a metering opening which has a sealing element to protect the interior of the reservoir from the environment, the reservoir being formed from a flexible film is known form DE-B-1288751. The known reservoir is provided with a flexible tube carrying a glass seal and a ring shaped screw-on connector for attaching to a drop counting device. After connection of the drop counting device, the glass seal can be broken and the liquid from the reservoir can be supplied to a patient via the drop counting device. The known construction has as a disadvantage that the relatively fragile glass seal may be broken inadvertently so that upon storage or transport of the reservoirs, leakage may occur. Furthermore, the known reservoir may not be emptied completely so that a residue may remain near the bottom part of the reservoir.

The aim of the present invention is to provide a system for the administration of a medical substance with which the abovementioned problems are overcome.

The aim of the present invention is, in particular, to provide a system with which risks, in particular with regard to contamination and spillage, can be limited, which is simple to handle, both when administering the substance to a patient and during sterilisation, which is flexible in use, production and storage and which gives rise to relatively little discarded material when being used.

To this end, the present invention proposes a system which is characterised in that the connecting section comprises: a coupling bush which is connected to the metering line system and can be detachably connected to the annular coupling element, the coupling bush being provided with an annular wall and a base surface having a central opening, the flexible channel section forming one end of the metering line system, extending through the opening in the coupling bush and terminating in a flexible sealing flange which is located in the coupling bush.

In this way it is possible first to connect the line system to the reservoir in the suitable manner in order only thereafter to connect the reservoir to the metering line system by breaking through the sealing element. The reservoir can thus be set up immediately in its operating position. The reservoir is frequently hung on a stand for this purpose. Only after the mechanical coupling between the reservoir and the metering line system has been properly connected is the reservoir opened, for example by puncturing. Because the flexible channel section terminates in a sealing flange, a reliable seal can be obtained when coupling the metering line system to the reservoir. During this operation the flexible material of the sealing flange is pushed, so as to produce a clamping effect, against the relatively rigid, for example annular, coupling element of the reservoir by the coupling bush. Because the flexible channel section is fed through the hole in the base of the coupling bush, said bush is able, during fixing thereof, to turn freely about the metering line, but the latter cannot be removed therefrom, so that an integral unit is formed. As a result, both the risk of spillage and the risk of contamination are severely restricted or even excluded. Because the reservoir is opened only after the metering line system has been reliably connected thereto, handling is also facilitated.

Because the line system is detachable from the reservoirs, said reservoirs can be produced, stored and transported in a simple manner without the line system forming an impediment thereto and without the risk of inadvertent opening of the reservoir by the penetration means. Furthermore, it is very simple for the medical staff to administer reservoirs of differing capacity to a patient, using a single metering line system. Furthermore, several reservoirs can be administered to a patient per treatment using a single metering line system. Consequently, the quantity of waste which is produced by the metering line systems is minimised. Finally, the detachable metering line systems and the reservoirs can be sterilised separately. Consequently it is possible, for example, to sterilize the metering line system by means of gamma radiation and to sterilise the flexible bags of the reservoir by means of high temperatures in an autoclave.

EP-A 0 258 579 discloses a system which has a reservoir that is provided with an annular coupling section to which a metering line system is detachably connected. A puncturing element in the metering line system can be pushed through the wall of the reservoir by compressing a flexible bellows. The connecting section between the reservoir and the metering line system is a simple snap connection with which an inadequately reliable seal can be obtained, so that the risk of leakage can arise. Furthermore, the coupling element is located on the reservoir in a position on the side wall, as a result of which it is possible for a relatively large volume of residual fluid to remain behind in the reservoir and/or in the connecting section. Consequently, the microbiological quality of the device is low.

To facilitate the deformation of the flexible channel section of the system according to the invention, said system can have a widened section. On the other hand, said channel section can be constructed with a thinner channel wall. The channel section can be made from material based on, for example, natural or synthetic rubber or silicones. Other appropriate materials which are easily elastically or plastically deformable and have a relatively low sensitivity to splitting on deformation, including metals and plastics, can also be suitable for said channel section. The penetration means can also be a different seal-breaking element and do not per se have to be an element which punctures the seal. For example, it is possible to fit the seal in a region which can easily be pinched, so that the seal automatically gives way on pinching. Firm pinching of, for example, the reservoir or other medium-filled chamber resulting in the seal giving way is also envisaged. Optionally, the rise in pressure caused by pinching, for example, the reservoir effects driving of the optional penetration means.

In one embodiment of a system according to the invention, the flexible sealing flanges and the flexible channel section are formed in one piece. As a result it is possible to make optimum use of the flexible characteristics of the flexible channel section. On the one hand, the channel section is used in order to break the sealing element on the reservoir and, on the other hand, it forms the flexible sealing flange which makes reliable sealing of the detachable metering line system possible.

The present invention relates both to a system composed of individual components and to an integrated system comprising a reservoir and a detachable metering line system. For example, it is possible for the manufacturer to supply the reservoir together with the metering line system as an integral whole, so that the nursing staff merely have to break the sealing element using the penetration means. The system is suitable for both sterilised and pasteurised products. Said products can be liquid nutrients, but also water, medicaments or blood. The reservoir can be a bag produced from flexible film, but, for example, can also be an essentially dimensionally stable plastic or glass bottle or otherwise. The construction of the reservoir is not important, as long the said reservoir is suitable for the purpose.

The invention also relates to a metering line system which is suitable for use with the system according to the invention, which metering line system is provided at one end with a flexible channel section, which can be shortened by deformation, and penetration means located in the channel section, which penetration means can be pushed through the sealing element of a reservoir on shortening the channel section, characterised in that the metering line system comprises a coupling bush which can be detachably connected to the annular coupling element of a reservoir, the coupling bush being provided with an annular wall and a base surface which has a central opening, the flexible channel section forming one end of the metering line system₁ extending through the opening in the coupling bush and terminating in a flexible sealing flange which is located in the coupling bush.

The invention also relates to a reservoir for use in a system according to the invention, which reservoir is provided with a metering opening which has a sealing element to protect the interior of the reservoir from the environment, which reservoir is formed from a flexible film, characterised in that the reservoir is provided with a relatively rigid, annular coupling element which is connected to the reservoir for detachable connection to the coupling bush of a line system, which coupling element is tubular and extends into the reservoir, a front and a back of the reservoir being joined in a liquid-tight manner to one another along two connection lines, which connection lines extend from the coupling element to the side edges of the reservoir and make an acute angle with the coupling element.

Preferably, the reservoir has a spout body or tube stump as coupling element, the end of which spout or tube stump carries the sealing element. On the one hand, a reliable connection between the reservoir and the metering line system can be achieved in this way. On the other hand, the sealing element can be reliably broken using the penetration means, especially when the penetration means are located on the connecting section or the metering line system.

The invention will be explained in more detail below with the aid of a non-limiting illustrative embodiment with reference to the drawings. In the drawings:
Fig. 1 shows a perspective view of a preferred embodiment of the system according to the invention;
Fig. 2 shows a side view, partially in cross-section, of a detail of the system of Fig. 1, shown in a first position; and
Fig. 3 shows a view corresponding to Fig. 2, which shows a second position.

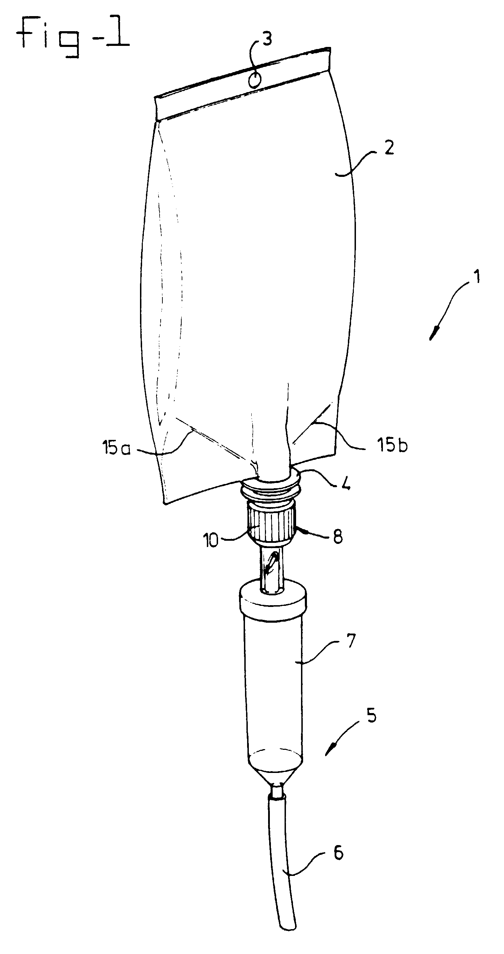

Fig. 1 shows the system 1. The figure shows a bag 2 of flexible film, which carries an eye 3 at its top for hanging the bag, in the position shown, on, for example, a stand (not shown). At its lower end, the bag 2 has a spout 4 to which a metering line system 5 is connected. The spout 4 is relatively rigid and inflexible. The tube 6 of said metering line system 5 runs in the known manner to the patient for enteral or parenteral administration of the contents of the bag 2.

In this embodiment the metering line system 5 also contains a conventional buffer 7, which can serve as a handle when fixing the metering line system 5 to the bag 2. The metering line system 5 is connected to the spout 4 by a coupling bush 10, which coupling bush 10 and spout 4 form part of the connecting section 8 for coupling the bag 2 to the metering line system 5.

The spout 4 of the bag 2 extends to within the bag. Preferably, the spout 4 is in the form of a tubular body in the bag and is provided with a number of feed openings in the tube wall inside the bag. The front and the back of the bag 2 are joined liquid-tight to one another in a V-shaped manner along join lines 15a and 15b, for example by sealing. Because the join lines 15a, 15b run at an acute angle with respect to the spout 4, a funnel is formed through which the residual fluid is fed from the bag to the lowest point of that part of the spout 4 which is located inside the bag 2. As a result, the amount of residual fluid which remains behind in the bag is minimised and a favourable microbiological climate is thus obtained.

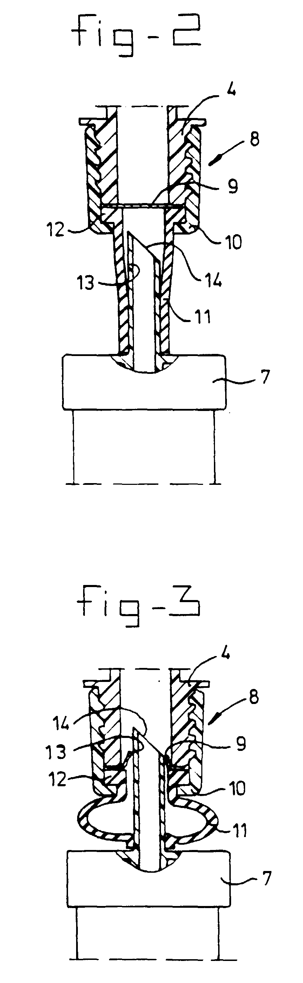

It can be seen in more detail from Figs 2 and 3 how the free end of the spout carries a sealing element or seal 9. Said seal 9 is made, for example, of a suitable foil-like material made, for example, of thin aluminium laminated with thin plastic. The connecting section 8 has a coupling bush, such as a screw top 10, which is screwed by its interior screw thread on the corresponding external thread on the end region of the spout 4. The top 10 has a central hole through which a channel section 11 protrudes. A flexible sealing flange 12 of the channel section 11 is enclosed between the top 10 and the spout 4 and thus forms a reliable medium-tight seal between the metering line system 5 and the bag 2. Said sealing flange 12 thus defines a packing element.

Hollow, cylindrical penetration means 13 are located inside the channel section 11. Said penetration means have a chamfered cutting face 14 at their top. The lower end of said means is attached to the housing of the buffer 7. The housing 7 thus also serves as a handle for the penetration means 13.

The channel section 11 is produced from relatively flexible material, for example silicone rubber. Said channel section 11 widens in the direction of the top 10. This is such that the channel section 11 closely surrounds the penetration means 13 at their base (by the buffer 7) and surrounds the penetration means 13 at their cutting face 14 with play. A medium-tight connection is ensured by the close abutment at the base.

As can be seen from Fig. 3, the penetration means 13 can be moved towards the seal 9 by deformation (elastic bulging out) of the channel section 11. As a result the penetration means 13 are capable of puncturing the seal 9. It will be clear that puncturing of the seal 9 has to take place only after the medium-tight connection between the bag 2 and the metering line system 5 has been achieved. The deformation of the channel section 11 is facilitated because said channel section 11 has a widened section. However, said widening is not essential for the functioning of the system.

Of course, the invention is not restricted to the embodiment described and shown here. For example, the penetration means 13 can be of different constructions; for example, said penetration means can be a solid needle which is accommodated in the channel section 11 with ample play. This likewise ensures that liquid flow is possible past or through said means 13. Furthermore, it is not important £or the channel section 11 to undergo a bulging-out deformation during puncturing of the seal 9 by the penetration means 13. Deformation involving bending or, for example, stretching of the channel section 11 are also possible options for other alternative embodiments. It is also not essential that the penetration means 13 are surrounded by the relatively easily deformable channel section 11; said channel section 11 could also be located elsewhere in the system. Furthermore, the penetration means 13 do not have to be located in the flow channel from the bag 2 to the metering line system 5, but can also be located in a branch thereof. In this way, the penetration means 13 extend, for example₁ into said flow channel only in order to break the seal 9. In addition, with this arrangement the penetration means 13 do not impede the free passage. However, from the standpoint of simplicity of production, an arrangement in the flow channel is to be preferred. Use in combination with a buffer 7 is also not essential.

The important feature is that an essentially medium-tight connection between the reservoir 2 and the metering line system 5 can first be obtained, after which the seal 9 is broken by the use of penetration means.

As a result of the relatively compact design of the connecting section 8 according to the present invention for connecting the bag 2 to metering line system 5, the flow characteristics of the fluid flowing through the connecting section are highly advantageous. The quantity of liquid which remains behind in the connection system after use of said system is relatively small. In this way an advantageous microbiological climate for the connecting section is obtained and risks of adverse effects as a consequence of residual fluid in the connecting section are minimised.

## Claims

1. System (1) for the administration of a medical substance, provided with a metering line system (5) for connection to a patient, a substance reservoir (2) which has a metering opening with a sealing element (9) to protect the interior of the reservoir from the environment and a connecting section (8) to connect the reservoir (2) via the metering opening to the metering line system (5), which connecting section defines a medium connection which is essentially hermetically screened from the environment when the sealing element is broken and comprises a flexible channel section (11), which can be shortened by deformation, penetration means (13), which are located in the channel section (11) and can be pushes through the sealing element (9) on shortening of the channel section and a relatively rigid coupling element (4), connected to the reservoir (2), characterised in that the connecting section comprises: a coupling bush (10) which is connected to the metering line system (5) and can be detachably connected to the coupling element (4), the coupling bush (10) being provided with an annular wall and a base surface having a central opening, the flexible channel section (11) forming one end of the metering line system, extending through the opening in the coupling bush and terminating in a flexible sealing flange (12) which is located in the coupling bush (10).

2. System according to Claim 1, characterised in that the flexible sealing flange (12) and the flexible channel section (11) are formed in one piece.

3. System according to Claim 1 or 2, characterised in that the end of the penetration means (13) terminates below the edge of the sealing flange (12), in the vicinity thereof.

4. System according to one of the preceding claims, characterised in that the flexible channel section (11) is no longer than 5 cm, preferably no longer than 2 cm, the penetration means (13) having a relatively narrow top section and a relatively broad base, which base is connected to a handle (7), the end of the flexible channel section (11) distal to the sealing flange (12) having a smaller internal diameter than the base of the penetration means (13) and being pushed over the latter so that it clamps against it.

5. System according to one of the preceding claims, wherein the coupling bush (10) is provided with an internal screw thread and the coupling element (4) of the reservoir is provided with an external screw thread.

6. System according to one of the preceding claims, characterised in that the penetration means (13) is a hollow needle which is provided with a slit, located in the longitudinal direction of the needle, which extends over the entire, relatively narrow top section of the needle.

7. Metering line system (5) for use in a system according to one of the preceding claims, which metering line system (5) is provided at one end with a flexible channel section (11), which can be shortened by deformation, and penetration means (13) located in the channel section (11), which penetration means (13) can be pushed through the sealing element (9) of a reservoir (2) on shortening the channel section (11), characterised in that the metering line system (5) comprises a coupling bush (10) which can be detachably connected to an annular coupling element (4) of a reservoir, the coupling bush (10) being provided with an annular wall and a base surface which has a central opening, the flexible channel section (11) forming one end of the metering line system, extending through the opening in the coupling bush (10) and terminating in a flexible sealing flange (12) which is located in the coupling bush.

8. Reservoir (2) for use in a system according to one of Claims 1 to 6, which reservoir is provided with a metering opening which has a sealing element (9) to protect the interior of the reservoir from the environment, which reservoir is formed from a flexible film, characterised in that the reservoir is provided with a relatively rigid, cylindrical coupling element (4) which is connected to the reservoir for detachable connection to the coupling bush of a metering line system, which coupling element is tubular and extends into the reservoir, a front and a back of the reservoir being joined in a liquid-tight manner to one another along two connection lines (151,156), which connection lines extend from the coupling element (4) to the side edges of the reservoir and make an acute angle with the coupling element.

## Patentansprüche

1. System (1) zur Verabreichung eines medizinischen Wirkstoffes, ausgestattet mit einem Meßleitungssystem (5) zum Anschluß an einen Patienten, einem Wirkstoffreservoir (2), welches eine Meßöffnung mit einem Dichtelement (9) zum Schutz des Inneren des Reservoirs von der Umgebung und einen Anschlußbereich (8) zur Verbindung des Reservoirs (2) über die Meßöffnung zu dem Meßleitungssystem (5) aufweist, wobei der Anschlußbereich eine Verbindung zum Medium definiert, welche im wesentlichen hermetisch von der Umgebung abgeschirmt ist, wenn das Dichtelement gebrochen ist und welches einen flexiblen Kanalbereich (11), der durch Verformung verkürzt werden kann, Eindringmittel (13), die in dem Kanalbereich (11) angeordnet sind und die durch das Dichtelement (9) durch Verkürzung des Kanalbereichs gestoßen werden können und ein relativ steifes Kupplungselement (4) umfaßt, welches mit dem Reservoir (2) verbunden ist, **dadurch gekennzeichnet**, daß der Anschlußbereich umfaßt: eine Kuppelhülse (10), welche mit dem Meßleitungssystem (5) verbunden ist und lösbar mit dem Kupplungselement (4) verbindbar ist, wobei die Kuppelhülse (10) mit einer Ringwand und einer Grundfläche mit einer zentralen Öffnung versehen ist, der flexible Kanalbereich (11) ein Ende des Meßleitungssystems bildet, welches sich durch die Öffnung in der Kuppelhülse erstreckt und in einem flexiblen Dichtungsflansch (12) endet, der in der Kuppelhülse (10) angeordnet ist.

2. System nach Anspruch 1, **dadurch gekennzeichnet**, daß der flexible Dichtungsflansch (12) und der flexible Kanalbereich (11) einstückig sind.

3. System nach einem der Ansprüche 1 oder 2, **dadurch gekennzeichnet**, daß die Eindringmittel (13) unterhalb der Kante des Dichtungsflansches (12) nahe diesem enden.

4. System nach einem der voranstehenden Ansprüche, **dadurch gekennzeichnet**, daß der flexible Kanalbereich (11) nicht länger als 5 cm ist, vorzugsweise nicht länger als 2 cm, die Eindringmittel (13) eine relativ enge Oberseite und eine relativ breite Unterseite aufweisen, wobei die Unterseite mit einer Halterung (7) verbunden ist, das Ende des flexiblen Kanalbereichs (11) distal zum Dichtungsflansch (12) einen kleineren Innendurchmesser hat als der Grund der Eindringmittel (13) und über diese geschoben ist, so daß es gegen diese klemmt.

5. System nach einem der voranstehenden Ansprüche, **dadurch gekennzeichnet**, daß die Kuppelhülse (10) mit einem Innengewinde versehen ist und daß das Kuppelelement (4) des Reservoirs mit einem Außengewinde versehen ist.

6. System nach einem der voranstehenden Ansprüche, **dadurch gekennzeichnet**, daß das Eindringmittel (13) eine Hohlnadel ist, welche mit einem Schlitz versehen ist, angeordnet in der longitudinalen Richtung der Nadel und sich über die gesamte, relativ enge Oberseite der Nadel erstreckt.

7. Meßleitungssystem (5) zur Verwendung in einem System gemäß einem der voranstehenden Ansprüche, wobei das Meßleitungssystem (5) an einem Ende mit einem flexiblen Kanalbereich (11) ausgestattet ist, welcher durch Verformung verkürzt werden kann, und Eindringmittel (13) in dem Kanalbereich (11) angeordnet sind, wobei die Eindringmittel (13) durch das Dichtelement (9) eines Reservoirs (2) unter Verkürzung des Kanalbereichs (11) gedrückt werden können, **dadurch gekennzeichnet**, daß das Meßleitungssystem (5) eine Kuppelhülse (10), welche lösbar mit einem ringförmigen Kupplungselement (4) eines Resevoirs verbindbar ist umfaßt, die Kuppelhülse (10) mit einer Ringwand und einer Grundfläche mit einer zentralen Öffnung versehen ist, der flexible Kanalbereich (11) ein Ende des Meßleitungssystems bildet, sich durch die Öffnung in der Kuppelhülse (10) erstreckt und in einem flexiblen Dichtungsflansch (12) endet, der in der Kuppelhülse angeordnet ist.

8. Reservoir (2) zur Verwendung in einem System gemäß einem der Ansprüche 1 bis 6, wobei das Reservoir mit einem Meßleitungssystem ausgestattet ist, welches ein Dichtelement (9) zum Schutz des Inneren des Reservoirs von der Umgebung aufweist, das Reservoir von einer flexiblen Folie gebildet ist, **dadurch gekennzeichnet**, daß das Reservoir mit einem relativ steifen, zylindrischen Kupplungselement (4) ausgestattet ist, welches mit dem Reservoir verbunden ist zur lösbaren Verbindung mit der Kuppelhülse eines Meßleitungssystems, wobei das Kuppelelement röhrenförmig ist und sich in das Reservoir erstreckt, eine Vorderseite und eine Rückseite des Reservoirs in flüssigkeitsdichter Weise entlang von zwei Verbindungslinien (151, 156) zusammengefügt sind, wobei die Verbindungslinien sich vom Kuppelelement (4) zu den Seitenkanten des Reservoirs erstrecken und einen spitzen Winkel mit dem Kupplungselement bilden.

## Revendications

1. Système (1) pour l'administration d'une substance médicale, muni d'un système de conduit de dosage (5) destiné à être connecté à un patient, d'un réservoir à substance (2) qui présente une ouverture de dosage avec un élément d'obturation (9) destiné à protéger l'intérieur du réservoir de l'environnement, et d'un segment de connexion (8) destiné à connecter le réservoir (2), par l'intermédiaire de l'ouverture de dosage, au système de conduit de dosage (5), segment de connexion qui définit une connexion de fluide qui est essentiellement isolée hermétiquement de l'environnement lorsque l'élément d'obturation est cassé et qui comprend un tronçon de conduit flexible (11) qui peut être raccourci par déformation, des moyens de pénétration (13) qui sont situés dans le tronçon de canal (11) et qui peuvent être poussés à travers l'élément d'obturation (9) lors du raccourcissement du tronçon de canal, et un élément de couplage (4) relativement rigide connecté au réservoir (2), caractérisé en ce que le segment de connexion comprend: une douille de couplage (10) qui est connectée au système de conduit de dosage (5) et qui peut être connectée à l'élément de couplage (4), la douille de couplage (10) étant pourvue d'une paroi annulaire et d'une surface de base présentant une ouverture centrale, le tronçon de conduit flexible (11) formant une extrémité du système de conduit de dosage s'étendant à travers l'ouverture dans la douille de couplage et se terminant par une bride d'obturation souple (12) qui est située dans la douille de couplage (10).

2. Système suivant la revendication 1, caractérisé en ce que la bride d'obturation souple (12) et le tronçon de conduit flexible (11) sont formés d'une seule pièce.

3. Système suivant la revendication 1 ou 2, caractérisé en ce que l'extrémité des moyens de pénétration (13) se termine au-dessous du bord de la bride d'obturation (12), à proximité de ce dernier.

4. Système suivant l'une des revendications précédentes, caractérisé en ce que le tronçon de conduit flexible (11) n'est pas plus long que 5 cm, de préférence pas plus long que 2 cm, les moyens de pénétration (13) présentant un segment supérieur relativement étroit et une base relativement large, base qui est connectée à une poignée (7), l'extrémité du tronçon de conduit flexible (11) distale par rapport à la bride d'obturation (12) présentant un diamètre intérieur plus petit que la base des moyens de pénétration (13) et étant poussée par-dessus cette dernière, de sorte qu'elle la serre.

5. Système suivant l'une des revendications précédentes, dans lequel la douille de couplage (10) est munie d'un filet intérieur et l'élément de couplage (4) du réservoir est muni d'un filet extérieur.

6. Système suivant l'une des revendications précédentes, caractérisé en ce que les moyens de pénétration (13) sont une aiguille creuse qui est pourvue d'une fente, située dans le sens longitudinal de l'aiguille, qui s'étend sur tout le segment supérieur relativement étroit de l'aiguille.

7. Système de conduit de dosage (5) destiné à être utilisé dans un système suivant l'une des revendications précédentes, système de conduit de dosage (5) qui est muni, à une extrémité, d'un tronçon de conduit flexible (11) qui peut être raccourci par déformation, et de moyens de pénétration (13) situés dans le tronçon de conduit (11), moyens de pénétration (13) qui peuvent être poussés à travers l'élément d'obturation (9) d'un réservoir (2) lors du raccourcissement du tronçon de conduit (11), caractérisé en ce que le système de conduit de dosage (5) comprend une douille de couplage (10) qui peut être connectée de manière amovible à un élément de couplage annulaire (4) d'un réservoir, la douille de couplage (10) étant pourvue d'une paroi annulaire et d'une surface de base présentant une ouverture centrale, le tronçon de conduit flexible (11) formant une extrémité du système de conduit de dosage s'étendant à travers l'ouverture dans la douille de couplage (10) et se terminant par une bride d'obturation souple (12) qui est située dans la douille de couplage.

8. Réservoir (2) destiné à être utilisé dans un système suivant l'une des revendications 1 à 6, réservoir qui est pourvu d'une ouverture de dosage présentant un élément d'obturation (9) destiné à protéger l'intérieur du réservoir de l'environnement, réservoir qui est formé à partir d'un film souple, caractérisé en ce que le réservoir est muni d'un élément de couplage cylindrique (4) relativement rigide qui est connecté au réservoir en vue d'une connexion amovible à la douille de couplage d'un système de conduit, élément de couplage qui est tubulaire et s'étend dans le réservoir, une partie avant et une partie arrière du réservoir étant assemblées l'une à l'autre de manière étanche aux liquides, le long de lignes de connexion (151, 156), lignes de connexion qui s'étendent de l'élément de couplage (4) aux bords latéraux du réservoir et forment un angle aigu avec l'élément de couplage.
